Europäisches Patentamt

European Patent Office

Office européen des brevets

(19)

(11) EP 0 777 742 B1

(12) **FASCICULE DE BREVET EUROPEEN**

(45) Date de publication et mention
de la délivrance du brevet:
**12.11.2003 Bulletin 2003/46**

(51) Int Cl.[7]: **C12Q 1/04**, B01J 20/28,
A61B 5/145, A61M 1/36,
A61M 5/31

(21) Numéro de dépôt: **96922958.2**

(22) Date de dépôt: **20.06.1996**

(86) Numéro de dépôt international:
**PCT/FR96/00955**

(87) Numéro de publication internationale:
**WO 97/000970 (09.01.1997 Gazette 1997/03)**

(54) **PROCEDE POUR ADSORBER DES AGENTS ANTI-MICROBIENS CONTENUS DANS UN LIQUIDE BIOLOGIQUE ET APPAREIL POUR LA MISE EN UVRE DE CE PROCEDE**

VERFAHREN ZUR ADSORPTION VON IN EINER BIOLOGISCHER FLÜSSIGKEIT ENTHALTENDEN ANTIMIKROBISCHEN AGENZIEN UND VORRICHTUNG ZUR DURCHFÜHRUNG DIESES VERFAHRENS

METHOD FOR ADSORBING ANTIMICROBIAL AGENTS CONTAINED IN A BIOLOGICAL FLUID, AND APPARATUS THEREFOR

(84) Etats contractants désignés:
**AT BE CH DE DK ES FI FR GB GR IE IT LI LU MC NL PT SE**

(30) Priorité: **22.06.1995 FR 9507739**

(43) Date de publication de la demande:
**11.06.1997 Bulletin 1997/24**

(73) Titulaires:
• **Institut francais du textile et de l'habillement 69130 Ecully (FR)**
• **BIO MERIEUX Société anonyme dite: F-69260 Charbonnieres les Bains (FR)**

(72) Inventeurs:
• **CHATELIN, Roger F-69380 Lissieu (FR)**
• **MONGET, Daniel F-01150 Saint-Sorlin-en-Bugey (FR)**

• **POLLET, Thierry F-69130 Ecully (FR)**
• **FITZER-COUTURIER, Catherine F-01120 Dagneux (FR)**
• **GAYRINE, Patrick F-69130 Ecully (FR)**

(74) Mandataire: **Vuillermoz, Bruno et al Cabinet Laurent & Charras B.P. 32 20, rue Louis Chirpaz 69131 Ecully Cédex (FR)**

(56) Documents cités:
EP-A- 0 105 579          EP-A- 0 231 105
EP-A- 0 517 189          EP-A- 0 597 542
EP-A- 0 628 346          FR-A- 2 543 849
US-A- 3 817 389          US-A- 4 174 277
US-A- 4 820 276

## Description

L'invention concerne un procédé pour adsorber ou éliminer des agents anti-microbiens, également dénommés "inhibiteurs" ou "agents inhibiteurs", contenus dans un liquide biologique, tel que notamment du sang ; elle concerne également un appareil pour la mise en oeuvre de ce procédé.

Dans la description et dans les revendications, par "agents anti-microbiens", "agents inhibiteurs" ou "inhibiteurs", on désigne tout agent destiné à éviter la prolifération des microbes ou des germes pathogènes, tels que par exemple des antibiotiques, des antifongiques, des antiviraux, des antiseptiques.

Par "liquide biologique", on désigne tout liquide contenant de la matière vivante, tel que du plasma, du liquide céphalorachidien, de l'urine, du liquide synovial, et de préférence du sang.

Dans la suite de la description, l'invention sera essentiellement illustrée à son application préférée au sang.

Pour de multiples applications, il est bien connu d'adsorber les antibiotiques contenus dans le sang, notamment en vue de les éliminer, pour analyser ensuite le sang ainsi épuré.

La détection des micro-organismes pathogènes dans les liquides biologiques, doit être effectuée dans des délais aussi courts que possible, notamment dans le cas de septicémie, pour lequel la mortalité reste élevée en dépit du large éventail d'antibiotiques dont disposent les médecins. Pour augmenter les chances de survie des malades, les praticiens administrent souvent aux patients un antibiotique ou un mélange d'antibiotiques. Il importe toutefois de déterminer une antibiothérapie appropriée dans les meilleurs délais. Malheureusement, l'isolement et la caractérisation rapide des germes infectieux est difficile lorsque les échantillons de sang à analyser contiennent des agents inhibiteurs, tels que des antibiotiques, qui souvent ralentissent, voire même suppriment la croissance microbienne.

Il en est de même avec les germes responsables des méningites, dont la présence dans le liquide céphalorachidien doit être détectée avec une extrême rapidité. Là également, cette mise en évidence peut être perturbée par la présence d'antibiotiques.

La bactériurie peut être également difficile à diagnostiquer lorsque le patient est placé sous antibiothérapie et excrète des antibiotiques dans l'urine. Fréquemment, l'isolement des germes infectieux peut être négatif.

Pour pallier ces inconvénients et améliorer la détection des microbes et leur isolement dans des échantillons de liquides biologiques de patients sous antibiothérapie, on a déjà proposé plusieurs méthodes visant à retirer ou à neutraliser les antibiotiques présents dans le sang, avant d'analyser le sang ainsi épuré.

On a tout d'abord proposé de faire passer le sang sur des résines échangeuses d'ions ou des polymères adsorbants. Pour ce faire, le sang prélevé chez le malade est mis dans un premier flacon contenant de telles résines ou de tels polymères et l'ensemble est légèrement agité pendant un temps approprié. Un volume de ce sang épuré est alors transféré dans un second flacon contenant le milieu de culture approprié pour la croissance des micro-organismes éventuellement présents. Cette technique permet une bonne adsorption de la plupart des antibiotiques. Toutefois, comme les matériaux adsorbants se présentent sous forme de granules ou de poudre indus dans les premiers flacons, cette technique extérieure au prélèvement du sang lui-même nécessite une étape supplémentaire de reprise et de transfert du sang ainsi épuré, laquelle est coûteuse, parfois d'une efficacité contestable, d'autant qu'on n'adsorbe pas tous les antibiotiques présents, et entraîne parfois des relargages dans le temps.

On a également suggéré de faire appel à des résines directement contenues dans des flacons d'analyse contenant le milieu de culture. Là également, on observe des risques de relargage des agents inhibiteurs dans le temps, et des risques d'inhibition dus à la composition même des résines.

Dans le document EP-A-0 597 542, correspondant au document US-A-5 314 855, on a proposé une composition adsorbante constituée d'un mélange de charbon actif, de terre de fuller, d'une résine en poudre échangeuse d'anions, et d'un mélange de polyélectrolytes cationique et anionique. Cette technique présente toutefois les mêmes inconvénients que ceux des résines échangeuses d'ions. En effet, il s'agit d'un procédé dans lequel le sang est tout d'abord prélevé, puis, en dehors du lieu de prélèvement, est simultanément traité et analysé dans le flacon d'analyse contenant un bouillon de culture approprié et la composition adsorbante.

En outre, puisque l'antibiotique reste dans le mélange, comme précédemment, on observe des risques de relargage. Par ailleurs, la présence de charbon actif en suspension perturbe l'analyse dans le flacon, car ce charbon peut adsorber d'autres composés, notamment des facteurs nécessaires à la croissance des micro-organismes, ce qui induit des résultats faussement négatifs. En outre, les surfaces d'échange s'avèrent souvent insuffisantes pour adsorber la totalité des agents inhibiteurs présents dans l'échantillon prélevé, de sorte que le flacon rempli doit être agité de manière significative pour obtenir une bonne efficacité d'échange. Enfin, cette technique du fait de la présence de charbon actif peut rendre les examens complémentaires difficiles, tels que notamment la lecture directe à l'oeil ou au microscope.

L'invention pallie ces inconvénients.

Elle vise un procédé d'adsorption, notamment des agents anti-microbiens contenus dans un liquide biologique, qui puisse être mis en oeuvre immédiatement de suite et en continu avec le prélèvement dudit liquide biologique et avant sa culture, ce qui évite toute interférence, et favorise la croissance des micro-organismes de l'échantillon à analyser.

Celà revêt une importance toute particulière, car lorsque les prélèvements sont effectués puis conservés dans une étuve pendant plusieurs heures en vue de réaliser une culture et une analyse subséquente, il peut se développer une action inhibitrice des agents antibiotiques sur les micro-organismes du prélèvement. Comme précédemment, cela peut induire des résultats faussement négatifs.

Selon le procédé de l'invention, pour adsorber des agents anti-microbiens contenus dans un liquide biologique, par passage de ce liquide sur un composé filtrant et adsorbant, ledit composé se présente sous forme d'une matière textile fibreuse à base de fibres de carbone activé et dépourvue de tout liant.

En d'autres termes, l'invention consiste à adsorber les agents anti-microbiens ou inhibiteurs contenus dans un liquide biologique, non plus à l'aide d'une résine échangeuse ou d'une composition à base de charbon actif et de terre de füller en grains ou en poudre, mais par passage d'un échantillon de sang immédiatement de suite après et en continu avec le prélèvement sur un matériau fibreux textile à base de fibres de carbone activé, dont la cohésion est assurée uniquement par leur entrelacement et donc en l'absence de tout liant, avant d'introduire le sang ainsi épuré dans les flacons contenant le milieu de culture, pour le développement des micro-organismes susceptibles d'être présents.

Comme on le sait, les fibres de carbone sont des matériaux qui présentent des caractéristiques mécaniques intéressantes, associées à une faible densité, qui permettent de les utiliser sous les formes textiles les plus variées, telles que filaments, fibres ou étoffes ou tresses, bi- ou tridimensionnelles. Le plus généralement, ces fibres sont fabriquées par pyrolyse d'un précurseur, notamment à base de fibres cellulosiques naturelles ou artificielles, voire synthétiques (fibres acryliques). Ces fibres de carbone étant bien connues, il n'y a pas lieu de les décrire ici en détail.

On a observé de manière totalement surprenante que pour obtenir le résultat recherché, à savoir l'adsorption des agents anti-microbiens, il importe que ces fibres de carbone aient été activées, c'est-à-dire aient subi une oxydation ménagée, qui provoque une attaque en surface et en profondeur de chacune des fibres, qui deviennent alors plus ou moins poreuses. Il se développe alors à la surface et dans le coeur de ces fibres des canaux plus ou moins radiaux, capables d'adsorber des molécules indésirables. Ainsi, plus les pores sont fins, plus la surface spécifique est importante et plus l'efficacité d'adsorption augmente. De même, il est possible de modifier les conditions d'oxydation des fibres en faisant varier notamment la température de traitement et ce, de manière à maîtriser la taille des pores développés à la surface et dans le coeur des fibres, et partant, leur sélectivité.

En pratique, on adapte la température d'oxydation de telle sorte à ménager au coeur et à la surface des fibres des pores de petites tailles, retenant ainsi les molécules indésirables tout en laissant passer les molécules de grandes tailles.

Le plus généralement, l'activation des fibres de carbone s'effectue par oxydation ménagée à une température comprise entre 800 et 1000°C en atmosphère inerte.

Les fibres de carbone activé sont bien connues dans le domaine de la désodorisation ou de l'épuration, notamment des gaz. Il est surprenant que l'utilisation de ces fibres de carbone activé permette également d'obtenir une telle efficacité et une telle rapidité d'adsorption, alors que les particules de charbon activé ou les fibres de carbone non activé ne permettent pas d'obtenir des résultats comparables, et surtout présentent des difficultés certaines de mise en oeuvre. De la sorte, l'homme de métier du domaine de l'analyse des agents microbiologiques connaissant les inconvénients précités de l'adsorption sur fibres de carbone ou sur particules de charbon activé, était dissuadé de faire appel à des fibres de carbone spécifiques, à savoir les fibres de carbone activé.

Par ailleurs, la cohésion des fibres de carbone activé est assurée par leur entrelacement, et ce en l'absence de tout liant, ce qui permet d'augmenter de façon surprenante la capacité de filtration et d'adsorption des fibres de carbone.

Grâce à un effet conjugué de rapidité et d'efficacité, le procédé selon l'invention devient compatible avec les vitesses de prélèvement du liquide biologique, et peut donc être effectué simultanément au prélèvement lui-même, et être incorporé ou intégré en continu dans le processus même de prélèvement, plus précisément entre le patient et en dehors du milieu analytique, évitant par là toute perturbation des analyses ultérieures.

Ainsi, la sélection de fibres de carbone activé autorise l'adsorption des agents anti-microbiens ou inhibiteurs immédiatement de suite et en continu avec le prélèvement, ce que l'on ne savait pas faire jusqu'alors. Celà se traduit par une simplicité dans la mise en oeuvre et une meilleure fiabilité des résultats obtenus.

Le matériau fibreux caractéristique de l'invention peut se présenter sous des formes les plus diverses. On peut faire appel par exemple à un faisceau de filaments parallèles. On peut également utiliser des fibres discontinues, des surfaces ou des étoffes textiles : tissées, tressées, tricotées, non tissées, bi- ou tridimensionnelles, feutres, bourres, voiles, velours, etc...

Il importe que la surface spécifique des fibres (ou des filaments) soit élevée. On a constaté que l'on obtient de bons résultats avec des surfaces spécifiques supérieures à 500 m$^2$/g, de préférence supérieures à 1000 m$^2$/g, pour un diamètre des fibres (ou des filaments) compris entre cinq et cinquante micromètres, de préférence voisin de dix micromètres.

Il importe que l'organisation de la matière fibreuse dans le dispositif permette le passage des grosses molécules ou de microorganismes du liquide biologique à analyser, mais permette de retenir des molécules plus petites, telles que les agents anti-microbiens ou inhibiteurs.

On peut ainsi faire passer le liquide à adsorber au contact des fibres ou des filaments organisés sous les formes ou surfaces évoquées ci-dessus. Ainsi, le matériau fibreux permet, d'une part, par la structure microporeuse des fibres de carbone activé d'assurer une bonne adsorption des agents anti-microbiens, et d'autre part, grâce à la structure du matériau lui-même, d'opérer une séparation sélective des constituants du liquide à analyser.

Selon la forme de présentation du matériau fibreux, le contact entre le liquide biologique s'effectue essentiellement en surface et/ou à coeur du matériau.

On a observé que l'on obtient de bons résultats en faisant écouler le liquide biologique dans un catheter souple de prélèvement à l'intérieur duquel on a inséré un faisceau de quelques (de cinq à cinquante) filaments parallèles de fibres de carbone activé.

Dans une autre forme de réalisation, le composé adsorbant conforme à l'invention est maintenu dans un module ou plusieurs modules superposés, que l'on intercale sur le trajet d'un catheter souple.

On obtient également de bons résultats en faisant écouler le liquide biologique à travers une seringue dont le corps cylindrique renferme un composé adsorbant du type de celui de l'invention.

L'efficacité du contact et de l'échange entre le liquide biologique et le composé filtrant et adsorbant conforme à l'invention, peut être avantageusement renforcée en disposant un répartiteur en amont dudit composé par rapport au sens de l'écoulement du liquide biologique, dont la fonction consiste à répartir le flux de liquide biologique sur toute la surface dudit composé filtrant et adsorbant.

L'écoulement peut également être effectué à travers des étoffes tridimensionnelles en forme de chicanes, de zig-zag, de spirales, ou autres pour augmenter le parcours et par là la surface et la durée, ainsi que l'efficacité du contact et de l'échange. On peut également utiliser des matériaux composites à structure ouverte (tricot à mailles lâches, structure cellulaire à pores ouverts).

Le choix de la forme de présentation du matériau fibreux proprement dit tient compte essentiellement du liquide à traiter, de la vitesse d'écoulement et des agents anti-microbiens à adsorber.

Le passage sur le matériau fibreux caractéristique à base de fibres de carbone activé, s'effectue par tous moyens connus. On peut faire appel à la gravité. De préférence, on fait passer le liquide sous une légère dépression, telle qu'une aspiration, suffisante pour attirer le liquide, à l'instar d'un prélèvement de liquide biologique sur un patient.

L'invention concerne également un appareil pour la mise en oeuvre de ce procédé. Cet appareil comprend :

- un moyen de prélèvement d'un liquide biologique ;
- un conduit, relié d'un côté au moyen de prélèvement et de l'autre côté à un récipient destiné à recevoir le liquide biologique extrait à analyser ;
- un moyen destiné à amorcer le prélèvement du liquide biologique.

Il se caractérise en ce que le conduit présente en son sein, et sur partie au moins de sa longueur, un matériau fibreux à base de fibres de carbone activé, au contact duquel passe le liquide biologique extrait au fur et à mesure du prélèvement.

En pratique :

- le moyen de prélèvement du liquide biologique est selon l'application envisagée, soit une aiguille (sang), soit une sonde (urine) ;
- le conduit est un catheter souple en une matière biocompatible, par exemple en matière plastique ;
- le récipient est associé au moyen pour amorcer le prélèvement ; ce peut être par exemple un flacon sous vide partiel ou un corps de pompe.

Dans une autre forme de réalisation, on fait s'écouler le liquide biologique à travers un ou plusieurs modules, chaque module définissant une cavité interne, et présentant de part et d'autre de ladite cavité deux ouvertures destinées à être emboitées, respectivement sur un conduit d'amenée et sur un conduit de sortie de liquide biologique.

En cas de pluralité de modules, ceux-ci sont superposés et dans tous les cas, chacun d'eux renferme une matière textile fibreuse à base de carbone activé occupant l'intégralité du volume disponible sur une épaisseur determinée.

Pour optimiser la répartition du flux de liquide biologique sur la surface de la matière textile fibreuse, chaque module renferme avantageusement un répartiteur disposé au moins en amont de la matière textile fibreuse, par rapport au sens d'écoulement du liquide biologique.

Préférentiellement, chaque module est de section transversale circulaire et la matière textile fibreuse à base de carbone activé se présente sous forme d'un disque de diamètre légèrement supérieur au diamètre interne de la cavité définie par le module, le disque occupant l'intégralité du volume disponible sur une épaisseur déterminée.

Par "diamètre légèrement supérieur", on désigne un diamètre supérieur de 1 à 2 millimètres au diamètre interne du module.

Enfin, le liquide biologique peut être également filtré au moyen d'une seringue, dont le corps cylindrique renferme une matière textile fibreuse conforme à l'invention, occupant l'intégralité du volume disponible sur une épaisseur déterminée. Dans cette forme de réalisation, le liquide à traiter est introduit dans le corps de la seringue, piston escamoté, puis il transite à travers la matière textile fibreuse après mise en place et translation du piston, avant d'être évacué par l'embout de fixation de l'aiguille ou l'aiguille elle-même.

Dans la description et dans les revendications, par l'expression "ocupant l'intégralité du volume disponible sur une épaisseur déterminée", on entend le volume disponible à l'intérieur du module ou du corps de la seringue, défini d'une part, par les parois internes latérales rspectivement du module et de la seringue, et d'autre part, par l'épaisseur déterminée conférée audit matériau, de telle sorte qu'il n'existe pas d'espace libre entre le matériau filtrant et adsorbant et lesdites parois.

La manière dont l'invention peut être réalisée et les avantages qui en découlent ressortiront mieux des exemples de réalisation qui suivent à l'appui de la figure unique annexée.

La figure 1 est une représentation schématique d'un appareillage conforme à l'invention.

La figure 2 est une coupe transversale d'un module conforme à l'invention.

La figure 3 est une représentation schématique d'une seringue conforme à l'invention.

*Appareil*

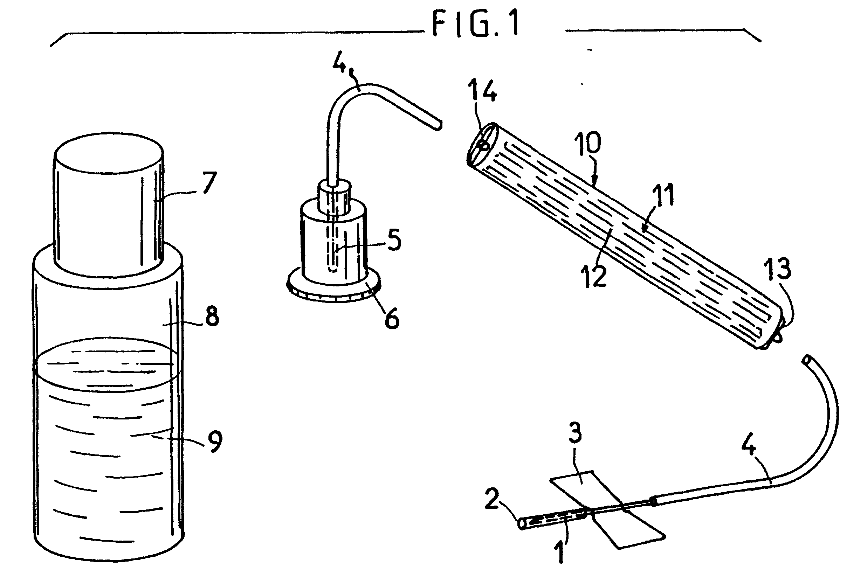

Un appareil de prélèvement et de traitement pour la mise en oeuvre de l'invention comprend (voir figure 1) de manière connue, respectivement une aiguille (1) à ailettes (3) placée de manière connue dans un tube de protection (2). L'extrémité de l'aiguille (1) est reliée à un catheter souple (4) connectée à son autre extrémité à une aiguille (5) entourée d'un capot de protection (6). Ce capot (6) est destiné à venir se positionner sur un bouchon (7), généralement en caoutchouc, d'un flacon (8) contenant un bouillon (9) de culture. A l'intérieur du flacon (8), règne une dépression correspondant à quelques centaines de Pa.

Selon l'invention, le catheter (4) est interrompu par un tube (10) avec lequel il communique, ledit tube contenant un faisceau (11) formé d'une pluralité de filaments parallèles (12) de carbone activé de dix micromètres de diamètre. Ce tube (10) est relié de manière connue par ses deux extrémités (13,14) au catheter (4).

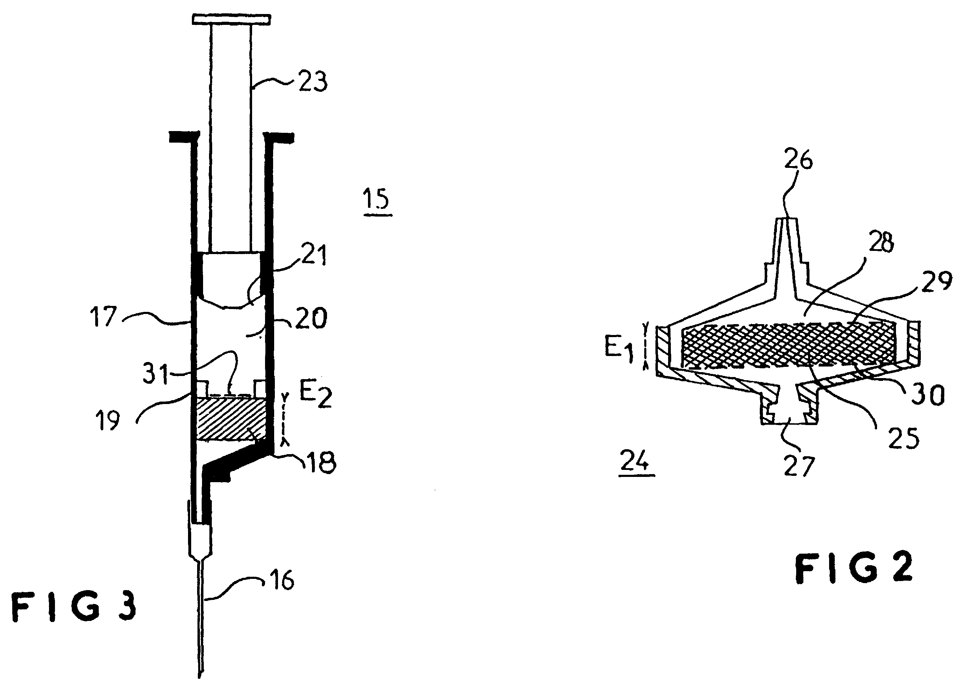

Avantageusement, on remplace le tube (10) par un module (24) muni de deux ouvertures, une ouverture femelle (26) recevant un conduit d'amenée de liquide biologique, et une ouverture mâle, prolongée par un canal (27) destiné à être emboité sur un conduit de sortie du liquide biologique (voir figure 2), ledit conduit de sortie de liquide biologique étant relié à un récipient destiné à recevoir le liquide biologique extrait à analyser. Ce module, de section transversale circulaire, délimite une cavité (28) renfermant deux répartiteurs (29, 30) disposés de part et d'autre d'une matière textile fibreuse à base de fibres de carbone activé, occupant l'intégralité du volume disponible sur une épaisseur déterminée (E1), les répartiteurs étant destinés à répartir le flux de liquide biologique sur la totalite de la matière textile. Ce module présente l'avantage de pouvoir être produit en grande quantité et de pouvoir s'adapter de façon universelle sur les catheters de prélèvement standard. En d'autres termes, ce dispositif permet d'épurer le sang immédiatement sur le lieu de prélèvement et ce de façon simple et économique.

Dans une forme de réalisation non représentée, on superpose une série de modules munis comme ci-avant d'une ouverture supérieure mâle et d'une ouverture inférieure femelle, destinées à être emboités respectivement sur les ouvertures des modules adjacents. Les ouvertures des modules ainsi définis sont également susceptibles d'être emboitées sur des conduits d'amenée et de sortie de liquide biologique.

Lorsqu'on veut analyser le sang d'un patient, on insère l'aiguille (1) dans une veine. Lorsque le sang apparaît dans le catheter (4), on place le capot (5) sur le bouchon (7) du flacon (8), en enfonçant l'aiguille (5) à travers le bouchon (7). La dépression du flacon (8) aspire le sang dans le circuit (4,10) qui s'écoule alors dans le flacon (8) où il réagit avec le bouillon (9).

Pendant ce prélèvement, le sang s'écoule sur et à travers le faisceau (11) de filaments parallèles de carbone activé qui retiennent alors les agents anti-microbiens ou inhibiteurs, puis le sang ainsi épuré est mis en contact avec le milieu de culture approprié, afin de permettre la croissance des micro-organismes éventuellement présents dans l'échantillon.

Dans une autre forme de réalisation (figure 3), l'appareil est constitué d'une seringue (15) comprenant de manière connue une aiguille (16), un corps cylindrique (17) et un piston (23). Le corps (17) comporte dans la partie immédiatement adjacente au canal d'adaptation de l'aiguille un feutre inséré en force, occupant l'intégralité du volume disponible sur une épaisseur déterminée (E2), formé d'une pluralité de filaments parallèles de carbone activé (18), maintenu par une bague d'arrêt (19) interdisant tout déplacement de celui-ci dans la seringue, ainsi que tout écoulement préférentiel, notamment le long des parois du corps. Par ailleurs, le volume (20) ménagé entre le matériau adsorbant de l'invention (18) et l'extrémité (21) du piston reçoit l'échantillon à traiter. En amont du feutre (18) par rapport au sens d'écoulement du liquide biologique, est disposé une grille (31) destinée à répartir le flux de liquide biologique sur la totalité de la surface dudit feutre.

Ainsi, lorsqu'on veut analyser le sang d'un patient, on retire le piston (23) de la seringue (15), puis on place l'échantillon à analyser dans le volume (20). On réintroduit ensuite le piston dans la seringue, puis par translation de celui-ci, on force le passage du liquide à travers le matériau adsorbant (18). L'échantillon est alors filtré, puis évacué par le canal d'adaptation de l'aiguille ou par l'aiguille elle-même, afin d'être mis au contact d'un milieu de culture approprié, dans un récipient indépendant.

*Validation du procédé de l'invention*

Le pouvoir d'adsorption vis-à-vis des antibiotiques est déterminé par la technique classique de diffusion en gélose décrite ci-après.

On prélève un échantillon de sang sur un patient normal et sain, c'est-à-dire exempt d'antibiotique. On ajoute ensuite dans ce sang une quantité prédéterminée d'un antibiotique majeur d'usage courant. On obtient ainsi un échantillon de référence, désigné ci-après S-ATB.

A l'aide d'une seringue, on prélève une partie de l'échantillon de ce sang S-ATB que l'on introduit dans le catheter (4) pour le faire passer sur la matière fibreuse (11).

L'échantillon de sang épuré tout comme l'échantillon de référence sont recueillis dans le bouillon (9). On laisse ensuite les hématies sédimenter pendant trente minutes dans des tubes à fond conique. On effectue ensuite un dosage bactériologique des antibiotiques.

De manière connue, on prélève une fraction de sang à analyser que l'on pose sur un disque de papier que l'on sèche. On place ensuite ce disque dans une boite de Petri à la surface de laquelle on dépose des bactéries sensibles à l'agent antimicrobien que l'on veut mettre en évidence et quantifier. On place ensuite la boite de Petri à l'étuve. Durant l'incubation, l'agent antimicrobien, s'il est présent, diffuse dans la gélose, et l'on obtient un diamètre d'inhibition après vingt-quatre heures.

On compare ensuite le diamètre obtenu à une gamme de référence pour pouvoir quantifier la quantité d'inhibiteurs présents, avant et après le traitement conforme à l'invention.

## Exemple 1

Dans cet exemple, le faisceau (11) est formé de huit brins de trente centimètres de longueur de fibres de carbone activé parallèles ayant un diamètre de dix micromètres, et présentant une surface spécifique de 1500 m$^2$/g. Le diamètre interne du tube (10) est de 3,10 mm.

La durée de passage d'un l'échantillon de cinq millilitres de sang est réglée par la dépression du flacon (8), ou par la dépression d'un piston, entre 15 et 45 secondes, pour correspondre à la durée d'un prélèvement normal de sang sur un patient.

La quantité d'antibiotiques adsorbée sur le matériau fibreux (11, 12), est mesurée par dosage classique microbiologique par diffusion en gélose sur boite de Petri. Le pourcentage d'inhibiteurs adsorbés sur le matériau fibreux est égal :

$$\frac{\text{valeur de la référence - valeur de l'essai}}{\text{valeur de la référence}} \times 100$$

Les résultats sont rassemblés dans le tableau I ci-après.

## Exemple 2

On répète l'exemple précédent en remplaçant le faisceau (12) par un faisceau de vingt-quatre brins identiques aux huit brins de l'exemple 1.

Les résultats sont rassemblés dans le tableau I ci-dessous.

| | Netilmicine | Vancomycine | Pefloxacine | Amoxicilline |
|---|---|---|---|---|
| Concentration en mg/l de l'agent antibiotique dans l'échantillon | 40 | 40 | 20 | 80 |
| Exemple 1 | 22 | 3 | 25 | 61 |
| Exemple 2 | 80 | 39 | 50 | 79 |

Les chiffres indiqués correspondent à des pourcentages d'adsorption pour le matériau fibreux (11, 12) caractéristique.

**Exemple 3**

On répète l'exemple 1 en remplaçant les fibres de carbone activé (12) caractéristiques de l'invention par des fibres de carbone de même titre et de même longueur, mais non activé.

On n'observe aucune adsorption.

**Exemple 4**

On répète l'exemple 1 en remplaçant les fibres de carbone activé par des grains de charbon actif.

On n'observe aucune adsorption significative.

Comme cela ressort de la description précédente, le procédé, l'appareillage et le dispositif selon l'invention permettent d'épurer le liquide biologique au fur et à mesure de son prélèvement.

De la sorte, l'invention peut être utilisée avec succès pour l'élimination des agents anti-microbiens ou inhibiteurs, du plasma, du liquide céphalorachidien, de l'urine, du liquide synovial, et surtout du sang.

**Revendications**

1. Procédé pour adsorber des agents anti-microbiens contenus dans un échantillon d'un liquide biologique, par passage de ce liquide sur un composé filtrant et adsorbant, *caractérisé* **en ce que** ledit composé se présente sous forme d'une matière textile fibreuse à base de fibres de carbone activé et dépourvue de tout liant.

2. Procédé selon la revendication 1, *caractérisé* **en ce que** le matériau fibreux se présente sous forme d'un faisceau (11) de filaments parallèles continus (12).

3. Procédé selon la revendication 2, *caractérisé* **en ce que** les filaments continus parallèles de carbone activé (12) présentent un diamètre compris entre cinq et cinquante micromètres.

4. Procédé selon la revendication 1, **caractérisé en ce que** la surface spécifique des fibres est supérieure à 500 m2/gramme.

5. Procédé selon la revendication 1, *caractérisé* **en ce que** le matériau fibreux se présente sous la forme de surfaces ou d'étoffes textiles : tissées, tressées, tricotées, non tissées, bi- ou tridimensionnelles, feutres, bourres, voiles, velours.

6. Procédé selon la revendication 5, *caractérisé* **en ce que** le matériau fibreux est une étoffe tridimensionnelle en forme de chicane, de zig-zag, de spirales.

7. Procédé selon la revendication 1, *caractérisé* **en ce que** le liquide biologique est du sang.

**8.** Appareil pour la mise en oeuvre du procédé selon l'une des revendications 1 à 7, du type comprenant :

- . un moyen de prélèvement (1) d'un liquide biologique ;
- . un conduit (4), relié d'un côté (13) au moyen de prélèvement (1) et de l'autre côté (14) à un récipient (9) destiné à recevoir le liquide biologique extrait à analyser ;
- . un moyen destiné à amorcer le prélèvement du liquide biologique;

*caractérisé* **en ce que** le conduit (4) est interrompue par un tube (10) avec lequel il communique, ledit tube (10) comprenant sur partie au moins de sa longueur, un matériau fibreux à base de fibres de carbone activé, au contact duquel passe le liquide biologique extrait au fur et à mesure du prélèvement.

**9.** Appareil selon la revendication 8, *caractérisé* **en ce que** le matériau fibreux est constitué d'un faisceau (11) de filaments continus parallèles (12) de fibres de carbone activé.

**10.** Appareil selon l'une des revendications 8 et 9, *caractérisé* **en ce que** le liquide biologique est du sang, et **en ce que** le conduit (4) est un catheter souple.

**11.** Appareil selon la revendication 8, *caractérisé* **en ce que** le récipient (8) destiné à recevoir le liquide biologique extrait à analyser est un flacon à l'intérieur duquel règne une dépression.

**12.** Dispositif pour adsorber des agents anti-microbiens contenus dans un liquide biologique, *caractérisé* **en ce qu'**il est constitué d'au moins un module (24), définissant une cavité interne (28), présentant de part et d'autre de ladite cavité, deux ouvertures (26, 27) destinées à être emboitées respectivement sur un conduit d'amenée et sur un conduit de sortie de liquide biologique, ladite cavité renfermant une matière textile fibreuse (25) à base de fibres de carbone activé, occupant l'intégralité du volume disponible au sein de la cavité sur une épaisseur déterminée.

**13.** Dispositif selon la revendication 12, *caractérisé* **en ce que** le module est de section transversale circulaire et **en ce que** la matière fibreuse à base de fibres de carbone activé se présente sous la forme d'un disque de diamètre légèrement supérieur au diamètre interne de la cavité définie par le module.

**14.** Dispositif pour adsorber des agents anti-microbiens contenus dans un liquide biologique, *caractérisé* **en ce qu'**il est constitué d'une pluralité de modules superposés selon les revendication 12 et 13, les deux ouvertures de chacun des modules étant susceptibles d'être emboitées sur les ouvertures respectives inférieures et supérieures des modules adjacents.

**15.** Dispositif selon l'une des revendications 12 à 14 *caractérisé* en ce chaque module renferme un répartiteur (29) disposé au moins en amont de ladite matière textile à base de fibres de carbone activé par rapport au sens d'écoulement du liquide biologique.

**16.** Dispositif pour adsorber des agents anti-microbiens contenus dans un liquide biologique, *caractérisé* **en ce qu'**il est constitué d'une seringue (15), dont le corps cylindrique (17) renferme une matière textile fibreuse (18) à base de fibres de carbone activé occupant l'intégralité du volume disponible sur une épaisseur déterminée.

**17.** Dispositif selon la revendication 16, *caractérisé* **en ce que** le corps cylindrique (17) de la seringue (15) renferme un répartiteur (31), disposé au moins en amont de ladite matière textile fibreuse (18) à base de fibres de carbone activé par rapport au sens d'écoulement du liquide biologique.

**Patentansprüche**

**1.** Verfahren zur Adsorption von in einer Probe einer biologischen Flüssigkeit enthaltenen Anti-Mikroben-Mitteln durch Leiten dieser Flüssigkeit über einen filtrierenden und adsorbierenden Verbundstoff, **dadurch gekennzeichnet, daß** der genannte Verbundstoff die Form eines faserigen textilen Materials auf Aktivkohlefaserbasis und ohne jegliches Bindemittel aufweist.

**2.** Verfahren nach Anspruch 1, **dadurch gekennzeichnet, daß** das Fasermaterial die Form eines Bündels (11) aus parallelen Filamentgarnen (12) aufweist.

3.  Verfahren nach Anspruch 2, **dadurch gekennzeichnet, daß** die parallelen Filamentgarne aus Aktivkohle (12) einen Durchmesser zwischen fünf und fünfzig Mikrometer aufweisen.

4.  Verfahren nach Anspruch 1, **dadurch gekennzeichnet, daß** die spezifische Oberfläche der Fasern 500 m$^2$/Gramm übersteigt.

5.  Verfahren nach Anspruch 1, **dadurch gekennzeichnet, daß** das Fasermaterial die Form von textilen Flächengebilden oder Stoffen mit folgenden Eigenschaften aufweist: gewebt, geflochten, gewirkt, Vliesstoff, zwei- oder dreidimensional, Filze, Scherwolle, Flor, Velours.

6.  Verfahren nach Anspruch 5, **dadurch gekennzeichnet, daß** das Fasermaterial ein dreidimensionaler Stoff in Labyrinthform, Zickzackform, Spiralform ist.

7.  Verfahren nach Anspruch 1, **dadurch gekennzeichnet, daß** die biologische Flüssigkeit Blut ist.

8.  Vorrichtung zur Durchführung des Verfahrens nach einem der Ansprüche 1 bis 7, des Typs, der folgendes umfaßt:

    - ein Mittel zur Entnahme (1) einer biologischen Flüssigkeit;
    - eine Leitung (4), welche an einer Seite (13) mit dem Mittel zur Entnahme (1) und an der anderen Seite (14) mit einem Behälter (9) verbunden ist, welcher zur Aufnahme der zu analysierenden abgenommenen biologischen Flüssigkeit bestimmt ist;
    - ein Mittel, welches dazu bestimmt ist, die Entnahme der biologischen Flüssigkeit einzuleiten;

    **dadurch gekennzeichnet, daß** die Leitung (4) durch eine Röhre (10) unterbrochen ist, mit der sie in Verbindung steht, wobei die genannte Röhre (10) mindestens auf einem Teil ihrer Länge ein Fasermaterial auf Aktivkohlefaserbasis umfaßt, mit dem die abgenommene biologische Flüssigkeit während der Entnahme in Berührung tritt.

9.  Vorrichtung nach Anspruch 8, **dadurch gekennzeichnet, daß** das Fasermaterial aus einem Bündel (11) aus parallelen Filamentgarnen (12) aus Aktivkohlefasern gebildet ist.

10. Vorrichtung nach einem der Ansprüche 8 und 9, **dadurch gekennzeichnet, daß** die biologische Flüssigkeit Blut ist und daß die Leitung (4) ein biegsamer Katheter ist.

11. Vorrichtung nach Anspruch 8, **dadurch gekennzeichnet, daß** der zur Aufnahme der zu analysierenden abgenommenen biologischen Flüssigkeit bestimmte Behälter ein Kolben ist, in dessen Inneren ein Unterdruck herrscht.

12. Vorrichtung zur Adsorption in einer biologischen Flüssigkeit enthaltener Anti-Mikroben-Mittel, **dadurch gekennzeichnet, daß** sie aus mindestens einem Modul (24) gebildet ist, welches einen inneren Hohlraum (28) definiert und welches auf beiden Seiten des genannten Hohlraums zwei Öffnungen (26,27) aufweist, die dazu bestimmt sind, auf eine Zufuhrleitung und eine Austrittsleitung für die biologische Flüssigkeit gesteckt zu werden, wobei der genannte Hohlraum ein faseriges textiles Material (25) auf Aktivkohlefaserbasis einschließt, welches die Gesamtheit des im Inneren des Hohlraums verfügbaren Raumes über eine bestimmte Dicke einnimmt.

13. Vorrichtung nach Anspruch 12, **dadurch gekennzeichnet, daß** das Modul einen kreisförmigen Querschnitt aufweist und daß das Fasermaterial auf Aktivkohlefaserbasis die Form einer Scheibe aufweist, deren Durchmesser ein wenig größer ist als der innere Durchmesser des durch das Modul definierten Hohlraums.

14. Vorrichtung zur Adsorption in einer biologischen Flüssigkeit enthaltener Anti-Mikroben-Mittel, **dadurch gekennzeichnet, daß** sie aus einer Mehrzahl übereinander angeordneter Module nach den Ansprüchen 12 und 13 gebildet ist, wobei die beiden Öffnungen jedes der Module auf die unteren bzw. oberen Öffnungen der benachbarten Module gesteckt werden können.

15. Vorrichtung nach einem der Ansprüche 12 bis 14, **dadurch gekennzeichnet, daß** jedes Modul einen Verteiler (29) einschließt, welcher mindestens stromaufwärts des genannten textilen Materials auf Aktivkohlefaserbasis in bezug auf die Strömungsrichtung der biologischen Flüssigkeit angeordnet ist.

16. Vorrichtung zur Adsorption in einer biologischen Flüssigkeit enthaltener Anti-Mikroben-Mittel, **dadurch gekennzeichnet, daß** sie aus einer Spritze (15) gebildet ist, deren zylindrischer Körper (17) ein faseriges textiles Material (18) auf Aktivkohlefaserbasis einschließt, welches die Gesamtheit des verfügbaren Raumes über eine bestimmte Dicke einnimmt.

**17.** Vorrichtung nach Anspruch 16, **dadurch gekennzeichnet, daß** der zylindrische Körper (17) der Spritze (15) einen Verteiler (31) einschließt, welcher mindestens stromaufwärts des genannten faserigen textilen Materials (18) auf Aktivkohlefaserbasis in bezug auf die Strömungsrichtung der biologischen Flüssigkeit angeordnet ist.

**Claims**

**1.** Method for adsorbing antimicrobial agents contained in a biological fluid, by passing this fluid over a filtering and adsorbing compound, *characterized* **in that** said compound takes the form of a fibrous textile material based on fibers of activated carbon and which is free from any binding agent.

**2.** Method according to claim 1, *characterized* **in that** the fibrous material takes the form of a bundle (11) of continuous parallel filaments (12).

**3.** Method according to claim 2, *characterized* **in that** the parallel continuous filaments of activated carbon (12) are between five and fifty micrometers in diameter.

**4.** Method according to claim 1, **characterized in that** the specific surface area of the fibers is greater than 500 m2/gram.

**5.** Method according to claim 1, *characterized* **in that** the fibrous material takes the form of surfaces or textile fabrics: woven, braided, knitted, nonwoven, two- or three-dimensional, felt, wadding, netting, velvet.

**6.** Method according to claim 5, *characterized* **in that** the fibrous material is a three-dimensional fabric in the form of twists and turns, zig-zags or spirals.

**7.** Method according to claim 1, *characterized* **in that** the biological fluid is blood.

**8.** Apparatus for carrying out the method according to one of claims 1 to 7, of the type comprising:

- . a means (1) for drawing a biological fluid;
- . a pipe (4), connected on one side (13) to the drawing means (1) and on the other side (14) to a vessel (8) designed to receive the extracted biological fluid to be analysed ;
- . a means designed to initiate the drawing of the biological fluid;

*characterized* **in that** the pipe (4) is interrupted by a tube (10) with which it communicates, said tube (10) comprising, over at least part of its length, a fibrous material based on fibers of activated carbon, in contact with which the extracted biological fluid passes as the drawing proceeds.

**9.** Apparatus according to claim 8, *characterized* **in that** the fibrous material consists of a bundle (11) of parallel continuous filaments (12) of fibers of activated carbon.

**10.** Apparatus according to either of claims 8 and 9, *characterized* **in that** the biological fluid is blood, and **in that** the pipe (4) is a flexible catheter.

**11.** Apparatus according to claim 8, *characterized* **in that** the vessel (8) designed to receive the extracted biological fluid to be analyzed is a bottle whose interior is at a reduced pressure.

**12.** Device for adsorbing antimicrobial agents contained in a biological fluid, *characterized* **in that** it consists of at least one module (24), defining an internal cavity (28), possessing, on each side of said cavity, two openings (26, 27) designed to be fitted, respectively, to an inflow pipe and to an outflow pipe for biological fluid, said cavity containing a fibrous textile material (25) based on fibers of activated carbon occupying the whole of the available volume within the cavity over a specified thickness.

**13.** Device according to claim *12, characterized* **in that** the module is of circular transverse section, and **in that** the fibrous material based on fibers of activated carbon takes the form of a disk of diameter slightly larger than the internal diameter of the cavity defined by the module.

14. Device for adsorbing antimicrobial agents contained in a biological fluid, *characterized* **in that** it consists of a plurality of superposed modules according to claims 12 and 13, the two openings of each of the modules being capable of being fitted to the respective lower and upper openings of the adjacent modules.

15. Device according to one of claims 12 to *14, characterized* **in that** each module contains a distributing device (29) arranged at least upstream of said textile material based on fibers of activated carbon, relative to the direction of flow of the biological fluid.

16. Device for adsorbing antimicrobial agents contained in a biological fluid, *characterized* **in that** it consists of a syringe (15), the cylindrical barrel (17) of which contains a fibrous textile material (18) based on fibers of activated carbon occupying the whole of the available volume over a specified thickness.

17. Device according to claim 16, *characterized* **in that** the cylindrical barrel (17) of the syringe (15) contains a distributing device (31) arranged at least upstream of said fibrous textile material (18) based on fibers of activated carbon, relative to the direction of flow of the biological fluid.

FIG.1

EP 0 777 742 B1

EP 0 777 742 B1

FIG 2

FIG 3

13